# EUROPEAN PATENT APPLICATION

(11) **EP 4 772 869 A1**
(43) Date of publication of application: **08.07.2026**
(21) Application number: 25214310.2
(22) Date of filing: 07.11.2025
(51) Int. Cl.: G01N 27/12, C01B 32/921, G01N 33/00, B82Y 30/00, B82Y 40/00

(54) **METHOD FOR MANUFACTURING OXIDIZED MXENE AND CHEMIMEMRISTOR DEVICE USING THE SAME**

(30) Priority: 07.01.2025 KR 20250002213
(71) Applicant: Industry-Academic Cooperation Foundation Gyeongsang National University, Jinju-si, Gyeongsangnam-do 52828 (KR)
(72) Inventor: PARK, Jun Hong, 52829 Jinju-si (KR)
(74) Representative: Caspary, Karsten

(57) **Abstract**

A method for manufacturing oxidized MXene according to a preferred embodiment of the present disclosure may remove fluorine groups (F) on a surface of MXene and form OH functional groups through an oxidation process to effectively adsorb CO₂ molecules and reversibly change electrical conductance according to gas concentration. In addition, a chemimemristor device based on the oxidized MXene (Mₙ₊₁Xₙ(OH)₂) may simultaneously implement memristor and gas sensor functions in a single device, and maintain multiple levels of conductance depending on the concentration of adsorbed molecules, and implement, based thereon, reversible changes in conductance according to a concentration of CO₂ and multi-level information processing through its characteristics of learning multi-level operations and molecular adsorption history to mimic the human sense of smell and synaptic plasticity of the brain.

## Description

### BACKGROUND OF THE INVENTION

### Field of the Invention

The present disclosure relates to a chemimemristor device, and more particularly, to a method for manufacturing oxidized MXene and a chemimemristor device using the same.

### Background of the Related Art

Existing semiconductor technology performs data processing and command execution based on a Von Neumann architecture. However, this structure cannot avoid a bottleneck phenomenon caused by continuous access between a central processing unit (CPU) and a memory, so it has limitations in processing large amounts of information. In order to solve such a problem, neuromorphic technology, which mimics the neuron structure of the human brain, is being actively developed. The neuromorphic technology may dramatically improve information processing efficiency by performing data recognition, perception, and computation in parallel. In particular, with the rapid development of AI and IoT technologies, the demand for low-power, high-intelligence semiconductor devices is increasing, and in order to achieve this, research on brain-mimicking semiconductor devices is being focused.

Conventional memory devices and gas detection sensors have been developed primarily to focus on a single function, while memristor-based devices are being utilized to implement neuromorphic systems through their electrical switching and nonvolatile memory characteristics. However, existing devices have limited expandability as multifunctional devices because they are insensitive to environmental factors or have insufficient capabilities to detect specific gas molecules. In particular, technological advancements are required to simultaneously implement gas sensing functions, reversible conductance changes resulting therefrom, and neuromorphic properties that mimic synaptic plasticity.

### SUMMARY OF THE INVENTION

The present disclosure is contrived to solve the foregoing problems, and an aspect of the present disclosure is to provide a manufacturing method for forming active reaction sites capable of adsorbing CO₂ gas molecules by oxidizing a surface of MXene. In addition, through this method, a chemimemristor device that maintains multiple states of conductance according to the concentration of adsorbed molecules based on oxidized MXene, and provides a learning function based on multi-level information processing and molecule adsorption history may be provided.

In order to achieve the technical tasks, a method for manufacturing oxidized MXene according to a preferred embodiment of the present disclosure may include etching a Mₙ₊₁AXₙ phase compound to manufacture Mₙ₊₁XₙF phase multilayer MXene (S01); and immersing the MXene in an aqueous hydrogen peroxide solution to form OH functional groups on a surface of the MXene through an oxidation reaction and then drying the same to manufacture oxidized MXene (Mₙ₊₁Xₙ(OH)₂) (S02).

Here, M is Ti, Zr, Hf, V, Nb, Ta, Cr, Mo, W or two or more transition metals thereof, A is Al, Ga, In, Tl, Si, Ge, Sn, Pb, P, As, Sb or a combination thereof, X is C, N or a combination thereof, and n is 1, 2 or 3.

In the step S02, through the oxidation reaction, an active reaction site capable of adsorbing CO₂ molecules may be formed.

In the step S02, forming, by the oxidation reaction, transition metal oxide particles represented by the chemical formula MO₂ on part of a surface of the oxidized MXene may be included.

A concentration of the aqueous hydrogen peroxide solution may be 0.01 wt% to 0.5 wt%.

In order to achieve the technical tasks, a chemimemristor device may include a gas sensing layer including oxidized MXene manufactured according to any one of claims 1 to 4, and having an active reaction site capable of adsorbing CO₂ molecules; and at least one conductive electrode in contact with a portion of the gas sensing layer.

The active reaction site may include carbon (C), titanium (Ti) and hydroxyl group (OH) sites of the oxidized MXene.

An order of high adsorption energy with CO₂ molecules at the active reaction site may be carbon (C), titanium (Ti), and hydroxy group (OH) sites.

An electrical conductance of the device may reversibly change according to a concentration of CO₂ gas.

The device may form a multi-level conducting state according to the concentration of CO₂ gas.

The device may have an expanded memory window and an increased ON/OFF ratio when exposed to CO₂ gas.

The device may operate as a neuromorphic system by mimicking short-term potentiation (STP) and short-term depression (STD) characteristics during CO₂ gas adsorption.

According to the present disclosure as described above, a method for manufacturing oxidized MXene according to a preferred embodiment of the present disclosure may remove fluorine groups (F) on a surface of MXene and form OH functional groups through an oxidation process to effectively adsorb CO₂ molecules and reversibly change electrical conductance according to gas concentration.

In addition, a chemimemristor device based on the oxidized MXene (Mₙ₊₁Xₙ(OH)₂) may simultaneously implement memristor and gas sensor functions in a single device, and maintain multiple levels of conductance depending on the concentration of adsorbed molecules, and implement, based thereon, reversible changes in conductance according to a concentration of CO₂ and multi-level information processing through its characteristics of learning multi-level operations and molecular adsorption history to mimic the human sense of smell and synaptic plasticity of the brain.

The effects of the present disclosure are not limited to those mentioned above, and may be clearly understood by those skilled in the art from the description throughout the specification, but also include other effects that are not explicitly mentioned.

### BRIEF DESCRIPTION OF THE DRAWINGS

FIG. 1 is a schematic diagram showing a method for manufacturing oxidized MXene according to Embodiment 1 of the present disclosure and a method for manufacturing a chemimemristor device using the same.
FIG. 2 is a result of confirming the microstructure and constituent elements of the oxidized MXene of Embodiment 1 and the MXene of Comparative Example 1, including FE-SEM images, EDS element mapping results, and TEM images and SAED pattern results of the MXene of Comparative Example 1 (a to c) and the oxidized MXene of Embodiment 1 (d to f).
FIG. 3 shows (a) SEM photographs, (b) XPS analyses, (c) XRD analyses, and (d) Raman spectra results of the oxidized MXene of Embodiment 1 and the MXene of Comparative Example 1.
FIG. 4 shows results of (a) I-V curve, (b) SET/RESET switching characteristics, (c) data retention time, (d) HRS and LRS conductance retention characteristics, and (e) short-term synaptic characteristics of a chemimemristor device of Embodiment 2 and a chemimemristor device of Comparative Example 2.
FIG. 5 shows (a) an I-V curve of the chemimemristor device of Embodiment 2 and the chemimemristor element of Comparative Example 2, and (b) an I-V curve of the chemimemristor device of Embodiment 2 depending on whether it is exposed to 25 ppm CO₂ gas.
FIG. 6 shows results of conductance change characteristics according to molecular triggering for the chemimemristor device of Embodiment 2.
FIG. 7 shows results of analyzing an interaction between oxidized MXene and CO₂ molecules through the density functional theory (DFT) for the chemimemristor device of Embodiment 2 and plane average charge density difference graphs thereof.
FIG. 8 shows results of comparing interaction characteristics and adsorption energy between oxidized MXene and CO₂ molecules for the chemimemristor device of Embodiment 2.
FIG. 9 shows a band structure subsequent to an interaction between oxidized MXene and CO₂ molecules for the chemimemristor device of Embodiment 2.
FIG. 10 shows results of projected density of states (DOS) for the chemimemristor device of Embodiment 2 before and after the adsorption of oxidized MXene and CO₂ molecules.
FIG. 11 shows results of (a) I-V curve, (b) data retention time, (c) short-term potentiation (STP), and (d) short-term depression (STD) for the chemimemristor device of Embodiment 2.
FIG. 12 shows results of (a) I-V characteristics according to CO₂ gas adsorption cycle, (b) I-V characteristics according to a concentration of CO₂, and (c) data retention characteristics (retention time) according to a concentration of CO₂ for the chemimemristor device of Embodiment 2.

### DETAILED DESCRIPTION OF THE PREFERRED EMBODIMENT

Hereinafter, preferred embodiments of the present disclosure will be described in detail with reference to the accompanying drawings. Advantages and features of the present disclosure, and methods of accomplishing the same will be clearly understood with reference to the following embodiments described in detail in conjunction with the accompanying drawings. However, the present disclosure is not limited to those embodiments disclosed below but may be implemented in various different forms. It should be noted that the present embodiments are merely provided to make a full disclosure of the invention and also to allow those skilled in the art to know the full range of the invention, and therefore, the present disclosure is to be defined only by the scope of the appended claims. Like reference numerals refer to like elements throughout the specification.

Unless otherwise defined, all terms (including technical and scientific terms) used herein may be used with meanings that can be commonly understood by those skilled in the art to which the present disclosure pertains. Additionally, terms defined in commonly used dictionaries are not interpreted ideally or excessively unless clearly specifically defined. It should be noted that the terms used herein are merely used to describe the embodiments, but not to limit the present disclosure. In this specification, unless clearly used otherwise, expressions in a singular form include a plural form.

The term "comprises" and/or "comprising" used in the specification intend to express a constituent element, a step, an operation and/or a device does not exclude the existence or addition of one or more other constituent elements, steps, operations and/or devices.

### Method for Manufacturing Oxidized MXene

A method for manufacturing oxidized MXene according to one embodiment of the present disclosure may perform etching a Mₙ₊₁AXₙ phase compound to manufacture Mₙ₊₁XₙF phase multilayer MXene (S01).

MXene, which is a two-dimensional material with conductivity, may be a film formed of a transition metal oxide or a transition metal nitride. Such a MXene film may be in a form of a plurality of MXene flakes or MXene nanosheets. For example, it may be a MXene nanosheet having multiple layers of several to several tens of layers.

The MXene nanosheets may be transition metal carbide or transition metal nitride represented by Mₙ₊₁XₙF (n=1, 2 or 3). Here, M, which is a transition metal, may include, for example, Ti, Zr, Hf, V, Nb, Ta, Cr, Mo, W, or two or more thereof, and X may be C, N, or a combination thereof.

The Mₙ₊₁XₙF (n=1, 2 or 3) may have a structure in which an atomic layer (X, specifically a carbon or nitrogen atomic layer) of carbon, nitrogen or a combination thereof is inserted between adjacent transition metal (M) atomic layers among two to four transition metal (M) atomic layers and covalently bonded to the transition metal. The transition metal atomic layer may include one transition metal or two or more different transition metals. Alternatively, the stacked transition metal atomic layers may be identical transition metal atomic layers or different transition metal atomic layers. Additionally, the stacked carbon or nitrogen (X) layers may all be carbon layers or nitrogen layers, or some layers may be carbon layers and the remaining some layers may be nitrogen layers.

A functional group having a negative charge, specifically F, may be located on a surface of the MXene nanosheet.

The Mₙ₊₁XₙF (n=1) may be Ti₂CF, V₂CF, Nb₂CF, Mo₂CF, Ti₂NF, V₂NF, Mo₂NF, (Ti_{0.5}Nb_{0.5})₂CF, (Ti_{0.5}V_{0.5})₂CF, or (Mo_{2/3}Y_{1/3})₂CF. The Mₙ₊₁XₙF (n=2) may be Ti₃C₂F, Ti₃CNF, Zr₃C₂F, Hf₃C₂F, (Ti_{0.5}V_{0.5})₃C₂F, (Cr_{0.5}V_{0.5})₃C₂F, (Cr₂/₃Ti_{1/3})₃C₂F, (Mo_{2/3}SC_{1/3})₃C₂F, Mo₂TiC₂F, or Cr₂TiC₂F. The Mₙ₊₁XₙF (n=3) may be Ti₄C₃F, V₄C₃F, Nb₄C₃F, Ta₄C₃F, (Nb_{0.8}Ti_{0.2})₄C₃F, (Nb_{0.8}Zr_{0.2})₄C₃F, (Mo_{0.5}Ti_{0.5})₄C₃F, or Mo₂Ti₂C₃F. Specifically, a MXene unit layer is preferably Ti₃C₂F.

Such a MXene nanosheet film, which is a material having a MAX phase, that is, a material having a structure in which a layer A, specifically an atomic layer A, is inserted between Mₙ₊₁Xₙ unit layers, may be obtained through a process of selectively etching the layer A. The A may be Al, Ga, In, Tl, Si, Ge, Sn, Pb, P, As, Sb or a combination thereof, and may be Al in one example. In this case, the etching process may be performed at approximately 40 to 80° C, specifically 50 to 70° C, using, for example, a hydrofluoric acid (HF) solution, and may be performed for 6 to 48 hours, specifically 12 to 24 hours, but is not limited thereto.

Then, immersing the MXene in an aqueous hydrogen peroxide solution to form OH functional groups data on a surface of the MXene through an oxidation reaction and then drying the same to manufacture oxidized MXene (Mₙ₊₁Xₙ(OH)₂) (S02) may be performed.

The oxidation step (S02) may be performed through a process of reacting MXene by adding it into an aqueous hydrogen peroxide solution.

A concentration of the aqueous hydrogen peroxide solution may be 0.01 wt% to 0.5 wt%. In this case, when the concentration is less than 0.01 wt%, a process time for oxidation of the surface of MXene may be added, or when the concentration exceeds 0.5 wt%, damage may occur due to rapid oxidation on the surface of MXene, which may increase the threshold voltage excessively. In this range, the uniformity of the surface chemical composition and oxidation reaction of MXene may be ensured. In one specific example, when the concentration of the aqueous hydrogen peroxide solution is 0.18 wt%, the removal of fluorine groups and the formation of OH groups on the surface of MXene may be effectively achieved, while the crystal structure of MXene may be stably maintained, but the present disclosure is not limited thereto, and an optimal oxidation conditions may be set by appropriately controlling the reaction time, temperature, and concentration according to the purpose.

In particular, the performing of the oxidation reaction to manufacture oxidized MXene may refer to forming an active reaction site capable of adsorbing CO₂ molecules on the surface of MXene. The aqueous hydrogen peroxide solution may change the chemical composition of the surface of MXene by removing the existing fluorine group (F) on the surface of MXene and forming a hydroxyl group (OH) in its place. Through this oxidation process, a layered structure of MXene is maintained, but a functional group on the surface may be changed to form a highly reactive active reaction site, or some carbon (C) sites may be exposed to the surface or a chemical bonding force of the surface may be reconstructed to form a state capable of strong bonding to CO₂ molecules.

In particular, in order to allow more effective adsorption of CO₂ molecules on the surface of MXene, the oxidized MXene manufactured through an oxidation reaction may have an active reaction site capable of adsorbing CO₂ molecules. In this case, the active reaction site includes carbon (C), titanium (Ti) and hydroxyl group (OH) sites of the oxidized MXene, and an order of high adsorption energy with CO₂ molecules at the active reaction site is carbon (C), titanium (Ti), and hydroxy group (OH) sites. Specifically, CO₂ is most stably adsorbed at the carbon (C) site, with an adsorption energy of 0.71 eV and a bonding distance of approximately 1.61 Å, which may form the strongest interaction. Next, at the titanium (Ti) site, relatively strong adsorption occurs with an adsorption energy of 0.68 eV and a bonding distance of 1.47 Å, but the adsorption stability may be formed somewhat lower than that of the carbon site. Finally, in the case of a hydroxyl group (OH) site, the adsorption energy is 0.19 eV and the bonding distance is 2.48 Å, which is the lowest value, which may mean that the interaction with CO₂ molecules is weak.

Therefore, the oxidation reaction may be said to be a key process by which oxidized MXene is converted into a high-performance material suitable for gas sensing and electrical property control.

Additionally, in the step S02, forming, by the oxidation reaction, transition metal oxide particles represented by the chemical formula MO₂ on part of a surface of the oxidized MXene may be included. Specifically, the step S02 may refer to chemically oxidizing the MXene to grow particle-shaped transition metal oxide nanocrystals on a surface of each layer of multilayer MXene nanosheets. In this case, functional groups (Tₓ) present on a surface of MXene are oxidized, and the oxidized functional groups and the transition metal (M) may be bonded to form transition metal oxide nanocrystals. The transition metal (M) may be an element derived from MXene. Transition metal oxide nanocrystal particles formed through the oxidation reaction may be uniformly formed on an interlayer and/or a surface of the MXene nanosheet, and a method such as ultrasonic treatment may be additionally and/or performed in parallel to uniformly locate them between the sheet layers. The transition metal oxide particles formed by the foregoing oxidation reaction are TiO₂, but are not limited thereto.

### Chemimemristor Using Oxidized MXene

The "chemimemristor" of the present disclosure is a next-generation memory device that, unlike an existing gasistor device, maintains multiple levels of conductance depending on the concentration of adsorbed molecules and allows multi-level operations based thereon. A chemimemristor device having the characteristic of storing a degree of molecular adsorption in a specific chemical environment and the resulting change in conductance in a memory may learn adsorption history to allow neuromorphic computing or data processing based on multiple states. The device has the characteristic of being able to process continuous and complex signals based on an electrochemical mechanism, compared to an existing gas-to-noise device that simply processes states of 1 and 0 based on molecular adsorption.

A chemimemristor device based on oxidized MXene according to one embodiment of the present disclosure may include a gas sensing layer including oxidized MXene manufactured by the foregoing method for manufacturing oxidized MXene; and at least one conductive electrode in contact with a portion of the gas sensing layer.

The gas sensing layer may be formed as a thin film by coating a solution including oxidized MXene manufactured by a method for manufacturing oxidized MXene. The solution including the oxidized MXene may use a method for coating in solution form on a silicon substrate on which a dielectric layer of several hundred nanometers in thickness is formed, or on a substrate on which a metal electrode, for example, a Pt thin film, is formed as a lower electrode on the silicon substrate.

The coating treatment may be performed without limitation using spin coating, dip coating, bar coating, and the like, thereby forming a thin film of oxidized MXene having a thickness ranging from several to several hundred nanometers.

The conductive electrode may be a metal or metal compound electrode having a thickness of several to several tens of nanometers formed on the oxidized MXene thin film, and may include a metal material such as Pt or Ag, and may use commonly used conductive materials, and may be selected based on electrical properties and ease of processing, which is not limited to a specific material.

The chemimemristor device may have a characteristic in which electrical conductance reversibly changes according to a concentration of CO₂ gas. This may be due to charge rearrangement that occurs during the adsorption and desorption process between OH functional groups formed on a surface of oxidized MXene and CO₂ molecules. As the concentration of CO₂ increases, CO₂ molecules are adsorbed at an active reaction site on the surface of the oxidized MXene to increase electrical conductance, and conversely, when CO₂ molecules are desorbed, the conductance of the device may be restored to its original state. Such a reversible change allows the device to function as a gas detection sensor with high sensitivity and reliability.

In addition, the chemimemristor device may exhibit a characteristic in which conductance improves in proportion to the concentration of CO₂ gas. This is because a change in conductance due to the adsorption of CO₂ molecules forms a specific resistance state (LRS/HRS), thereby implementing electrical hysteresis characteristics. Therefore, the chemimemristor device may be utilized as a multi-functional device that combines data storage and information processing functions while quantitatively detecting gas concentration. In addition, the device may process multi-state information by continuously changing the resistance state according to the gas concentration, and may also be utilized as a neuromorphic system by mimicking synaptic plasticity such as short-term potentiation (STP) and short-term depression (STD).

In addition, the chemimemristor device may exhibit characteristics in which a memory window expands and an ON/OFF ratio increases when exposed to CO₂ gas. This is because the adsorption of CO₂ molecules strengthens the charge transfer and electrical conductance change on a surface of oxidized MXene, and as the concentration of gas increases, a difference between resistance states (LRS/HRS) of the device becomes more pronounced, and thus the ON/OFF ratio may be improved. These characteristics mean that the chemimemristor device can maximize its performance as a gas detection sensor while simultaneously implementing its electrical hysteresis characteristics as a memristor device in a more stable and extended form.

The chemimemristor device operates as a neuromorphic system by mimicking short-term potentiation (STP) and short-term depression (STD) characteristics during CO₂ gas adsorption. This is because CO₂ molecules induce reversible changes in electrical conductance during the adsorption and desorption process at active reaction sites on the surface of oxidized MXene, and these characteristics are similar to neuromorphic behaviors that implement short-term memory formation and plasticity of biological synapses. Therefore, the device of the present disclosure may be utilized for gas detection as well as learning and information processing of neural networks in low-power based artificial intelligence (AI) and neuromorphic computing systems.

Hereinafter, the present disclosure will be described in more detail using embodiments and comparative examples. However, the following embodiments and comparative examples are intended to illustrate the present disclosure, and the scope of the present disclosure is not limited thereto.

### Embodiment 1: Manufacture of Oxidized MXene

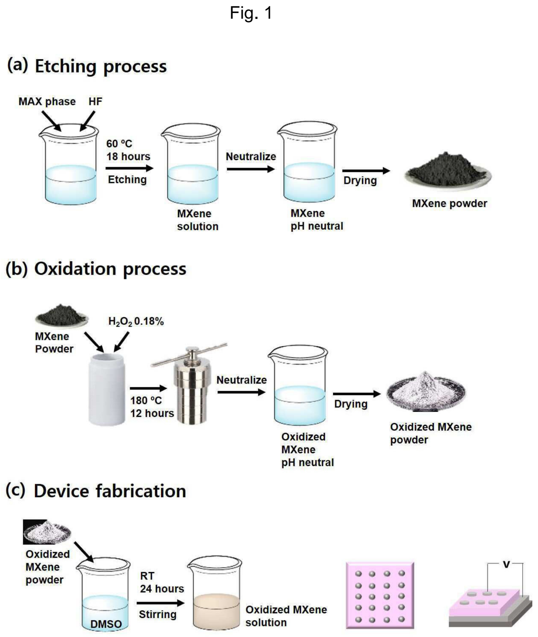

FIG. 1 is a schematic diagram showing a method for manufacturing oxidized MXene according to Embodiment 1 of the present disclosure and a method for manufacturing a chemimemristor device using the same.

Referring to FIG, 1, the manufacture of oxidized MXene (Ti₃C₂(OH)₂) is performed by an etching process of first reacting MAX phase (Ti₃AlC₂) with an HF solution, etching at 60° C for 18 hours to obtain a Ti₃C₂F phase MXene solution, and neutralizing and drying the MXene solution to acquire MXene powder. Then, the MXene powder is added to a 0.18% H₂O₂ aqueous solution and oxidized at 180° C for 12 hours so as to perform an oxidation process of removing fluorine groups (F) on the surface, forming OH functional groups, and generate some TiO₂. Next, the dried oxidized MXene powder is mixed with a dimethyl sulfoxide (DMSO) solvent and stirred at room temperature for 24 hours to manufacture an oxidized MXene solution.

### Comparative Example 1: Manufacture of Non-oxidized MXene

MAX phase (Ti₃AlC₂) was reacted with HF solution and etched at 60° C for 18 hours to manufacture a MXene solution.

### Embodiment 2: Manufacture of Chemimemristor Device Based on Oxidized MXene

The MXene oxide solution of Embodiment 1 was coated on a lower electrode, then an upper electrode was deposited to manufacture an oxidized MXene-based chemimemristor device (device fabrication).

### Comparative Example 2: Manufacture of Gasistor Device Based on Non-oxidized MXene

A gasistor device based on MXene was manufactured using the same method as in Embodiment 1, except that the MXene solution of Comparative Example 1 was used.

### Experimental Example 1: Evaluation of Characteristics of Oxidized MXene in Embodiment 1 and MXene in Comparative Example 1

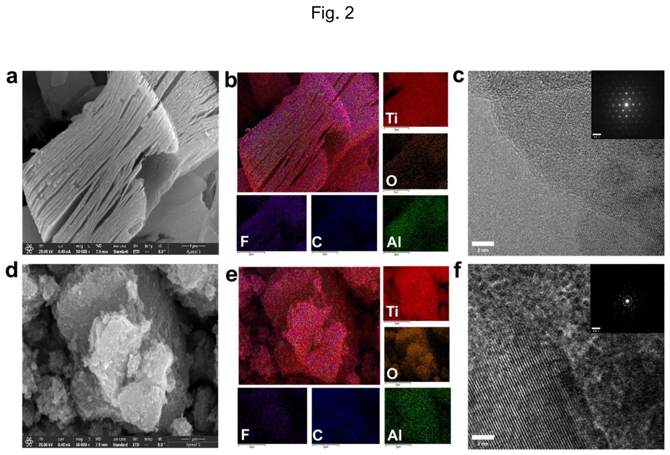

FIG. 2 is a result of confirming the microstructure and constituent elements of the oxidized MXene of Embodiment 1 and the MXene of Comparative Example 1, including FE-SEM images, EDS element mapping results, and TEM images and SAED pattern results of the MXene of Comparative Example 1 (a to c) and the oxidized MXene of Embodiment 1 (d to f).

Referring to FIG. 2, it can be seen in (a) of FIG. 2, which is an FE-SEM image of MXene, that it has a typical smooth and dense layered structure, and an EDS element mapping in (b) of FIG. 2 shows a uniform distribution of Ti, O, F, C, and Al present on the surface. A TEM image and SAED pattern in (c) of FIG. 2 show a high crystallinity of MXene. In contrast, a FE-SEM image of oxidized MXene in (d) of FIG. 2 shows an irregular and granular surface after an oxidation process, which is confirmed to be a structural change due to the formation of TiO₂ and the introduction of OH groups. An EDS element mapping in (e) of FIG. 2 confirms a decrease in fluorine (F) and an increase in oxygen (O), which is a result of the removal of fluorine groups and the formation of OH groups. A TEM image and SAED pattern in (f) of FIG. 2 show that irregular particles and TiO₂ are formed on a surface of oxidized MXene, while some crystallinity is maintained.

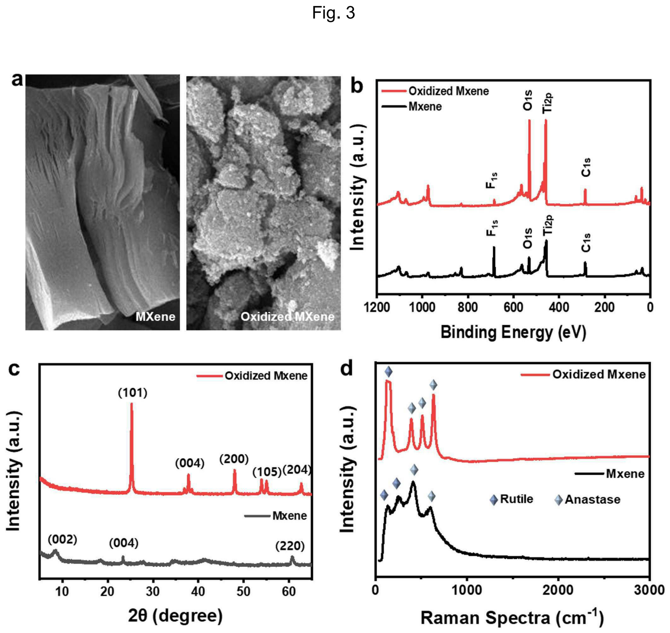

FIG. 3 shows (a) SEM photographs, (b) XPS analyses, (c) XRD analyses, and (d) Raman spectra results of the oxidized MXene of Embodiment 1 and the MXene of Comparative Example 1.

Referring to FIG. 3, it can be seen in an FE-SEM image in (a) of FIG. 3 that MXene exhibits a smooth and dense layered structure, while oxidized MXene has titanium oxide (TiO₂) formed on the surface after the oxidation process. In an XPS spectrum in (b) of FIG. 3, it can be seen that titanium oxide was formed on a surface of MXene by increasing a peak intensity of oxygen (O₁ₛ) in oxidized MXene. An XRD pattern in (c) of FIG. 3 shows that the characteristic peaks of MXene coincide with those of the structure of Ti₃C₂Tₓ, and additional diffraction peaks appear in the case of oxidized MXene, and it can be seen that they coincide with those of the titanium oxide (TiO₂) phase. Finally, in a Raman spectrum in (d) of FIG. 3, the peaks of MXene coincide those of Ti₃C₂Tₓ, and in oxidized MXene, additional peaks of TiO₂ phase (Rutile and Anatase) appear along with the MXene characteristics. Through this, it can be seen that fluorine groups (F) on a surface of MXene were removed through the oxidation process, and titanium oxide was formed as oxygen was introduced.

### Experimental Example 2: Characteristics of Chemimemristor Device Based on Oxidized MXene Expressed by CO₂ Molecules

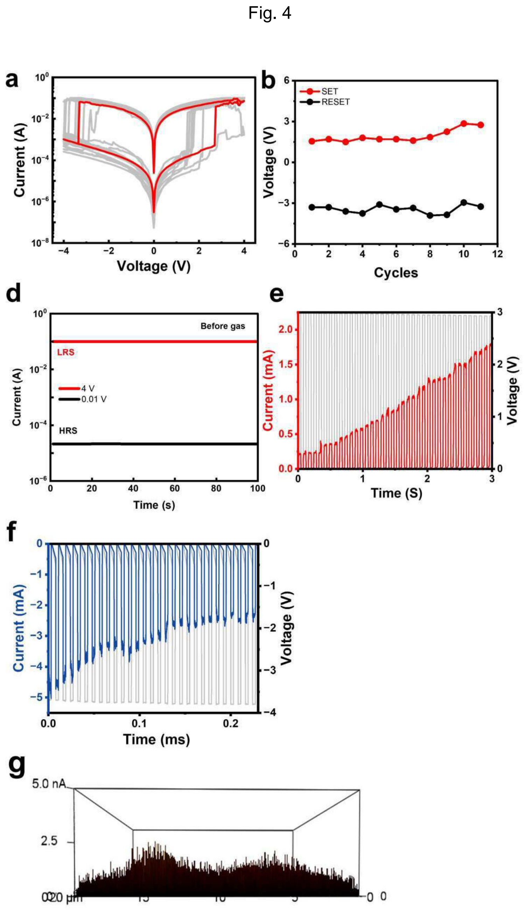

FIG. 4 shows results of (a) I-V curve, (b) SET/RESET switching characteristics, (c) data retention time, (d) HRS and LRS conductance retention characteristics, and (e) short-term synaptic characteristics in a chemimemristor device of Embodiment 2 and a gasistor device of Comparative Example 2.

Referring to FIG. 4, it can be seen that an oxidized MXene device exhibits reversible SET/RESET switching characteristics through I-V characteristics, and operates as a memristor device. Through (b) of FIG. 4, SET and RESET voltages are maintained steadily even in repeated cycle tests, and it can be seen that they have a cyclic stability of the device, and as a result of analyzing a data retention time of the device before the exposure to CO₂ gas in (c) of FIG. 4, it can be seen that a high resistance state (HRS) and a low resistance state (LRS) are stably maintained for a long period of time. Additionally, in (d) and (e) of FIG. 4, the oxidized MXene device exhibited neuromorphic properties that mimic synaptic plasticity through short-term potentiation (STP) and short-term depression (STD) characteristics. These results indicate that an oxidized MXene-based chemimemristor device can be utilized in a neuromorphic system and gas sensing device through synaptic plasticity while implementing stable memristor characteristics.

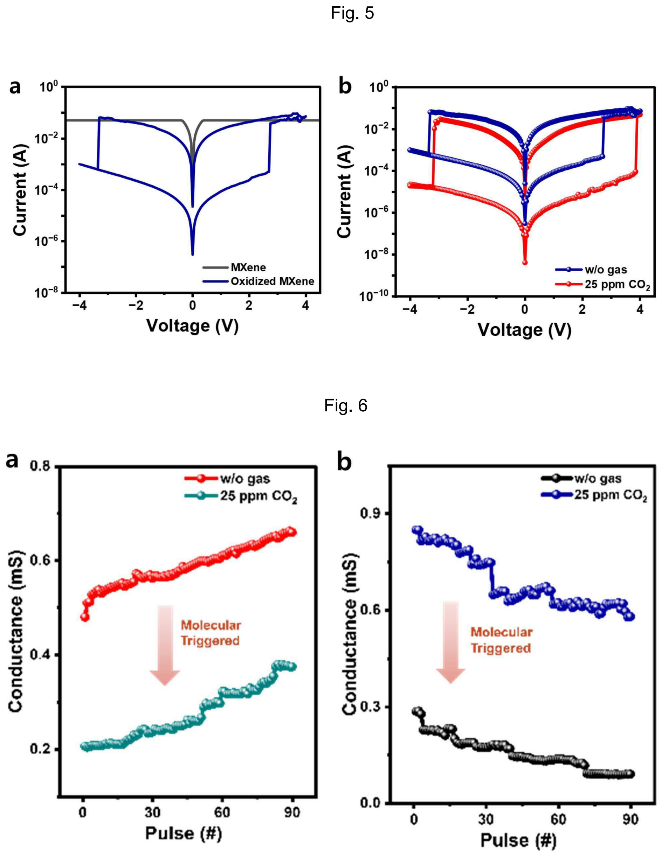

FIG. 5 shows (a) an I-V curve of the chemimemristor device of Embodiment 2 and the gasistor element of Comparative Example 2, and (b) an I-V curve of the chemimemristor device of Embodiment 2 depending on whether it is exposed to 25 ppm CO₂ gas.

Referring to FIG. 5, results of comparing the electrical properties of the chemimemristor device, and analyzing the performance change of the oxidized MXene device according to the exposure to CO₂ gas are shown. (a) of FIG. 5 shows an I-V curve of a chemimemristor device using oxidized MXene of Embodiment 1 and a gasistor device using non-oxidized MXene of Comparative Example 1, and it can be seen that when oxidized MXene is applied, the device has memory characteristics with an improved ON/OFF ratio to approximately 10². It can be seen that TiO₂ phase formed on a surface of MXene contributed to the conductance and hysteresis characteristics of the device. (b) of FIG. 5 shows I-V curves in which oxidized MXene-based chemimemristor devices manufactured in Embodiment 1 are compared before and after the exposure to 25 ppm CO₂ gas. When exposed to CO₂ gas, an ON/OFF ratio of the oxidized MXene device increases from approximately 10² to 10³, and it can be seen that a memory window is expanded. These results indicate that CO₂ gas adsorption changes charge transfer characteristics on a surface of oxidized MXene, thereby improving the conductance of the device and maximizing memory characteristics. Through this, it can be seen that the basic memory characteristics of the oxidized MXene-based chemimemristor device are improved by the formation of the TiO₂ phase, and I-V characteristics are further improved by the exposure to CO₂ gas.

FIG. 6 shows results of conductance change characteristics according to molecular triggering for the chemimemristor device of Embodiment 2.

Referring to (a) of FIG. 6, conductance changes before and after the exposure to 25 ppm CO₂ gas are shown. When CO₂ gas is not present (red), the conductance of the device tends to increase steadily, but when exposed to 25 ppm CO₂ gas (cyan), the conductance decreases significantly due to molecular triggering. This indicates that the conductive properties of the device have changed as CO₂ molecules interacted with the surface of oxidized MXene. In addition, referring to (b) of FIG. 6, a conductance change according to the exposure to CO₂ gas in a vacuum (before the injection of CO₂: 1 x 10⁻² torr) condition at 25° C is shown. In the absence of gas (black), the conductance of the device tends to decrease steadily, but in the state exposed to 25 ppm CO₂ gas (blue), the conductance is observed to remain relatively high due to molecular triggering.

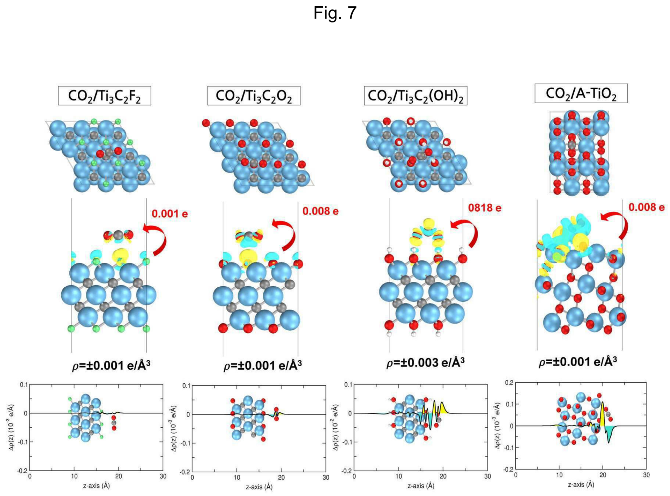

FIG. 7 shows results of analyzing an interaction between oxidized MXene and CO₂ molecules through the density functional theory (DFT) for the chemimemristor device of Embodiment 2 and plane average charge density difference graphs thereof.

Referring to FIG. 7, it shows a structural site where CO₂ is adsorbed to different oxidized MXene structures (Ti₃C₂F₂, Ti₃C₂O₂, Ti₃C₂(OH)₂, A-TiO₂). In each structure, the adsorption location of CO₂ molecules and the resulting charge redistribution may be identified, where the yellow and blue areas represent the accumulation (positive) and loss (negative) of charge, respectively. In the structure of Ti₃C₂F₂, an interaction with CO₂ was weak, resulting in a minimal charge transfer (0.001e), whereas in the structures of Ti₃C₂O₂ and Ti₃C₂(OH)₂, charge transfer increased significantly to 0.008e and 0.818e, respectively, due to a strong interaction with CO₂. In particular, it can be seen that the structure of Ti₃C₂(OH)₂ is strongly bonded to CO₂ to induce greater charge transfer. Although the adsorption of CO₂ was observed in A-TiO₂, charge transfer was 0.008e, which was relatively low compared to Ti₃C₂(OH)₂. In addition, plane average charge density difference graphs at the bottom show a change in charge density in a z-direction between CO₂ and oxidized MXene. The structure of Ti₃C₂(OH)₂ shows a largest charge density difference, indicating that charge redistribution between the surface and molecules occurs actively due to strong bonding with CO₂.

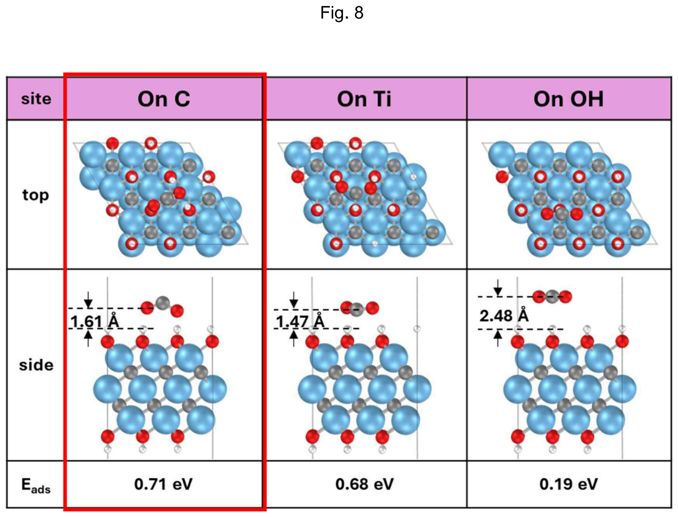

FIG. 8 shows results of comparing interaction characteristics and adsorption energy between oxidized MXene and CO₂ molecules for the chemimemristor device of Embodiment 2.

Referring to FIG. 8, a structure of CO₂ molecules bonded to each adsorption site can be seen through top and side views, and adsorption energy (E_{ads}) and a bonding distance can be compared. CO₂ was most strongly adsorbed at the carbon (C) site, thus exhibiting a highest value with a bonding distance of 1.61 Å and adsorption energy of 0.71 eV, and showing a most stable adsorption state. At the titanium (Ti) site, the adsorption energy was 0.68 eV and the bonding distance was 1.47 Å, thus showing a relatively strong interaction, but the adsorption energy was found to be lower than that of the carbon site. Although the Ti site can also interact with CO₂, Ti atoms have relatively stronger metal-oxygen bonds, so the bond with the oxygen of CO₂ may have slightly lower adsorption energy than the carbon site. Meanwhile, at the hydroxyl group (OH) site, the adsorption energy is the lowest at 0.19 eV and the bonding distance is also long at 2.48 Å, indicating that a weak bond is formed. The OH site has a very weak interaction with CO₂, because the OH group is already bonded with hydrogen and has low reactivity. Prior to the oxidation process, fluorine groups (F) were adsorbed on a surface of MXene due to the etching process using HF, which inhibited the reaction with CO₂ molecules, but as the oxidation process progresses, fluorine ion (F⁻) groups are desorbed (removed), and through this, the surface of MXene is activated, allowing CO₂ molecules to be bonded with relatively high bonding energy.

Through this, it can be seen that CO₂ is stably adsorbed mainly at carbon sites. This result is likely due to the fact that the carbon site provides the most energetically stable adsorption state through electronic interaction and van der Waals bonding with CO₂, and the removal of fluorine groups (F) during the oxidation reaction process may be the main cause of the improved activation and structural accessibility of the carbon site. Therefore, the carbon site of the oxidized MXene acts as the most important active reaction site for CO₂ detection, and may exhibit high gas detection performance and electrical characteristic changes of the chemimemristor device.

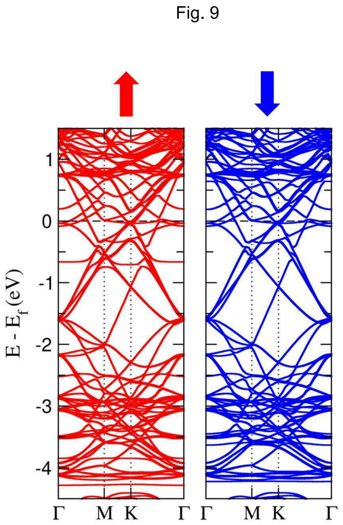

FIG. 9 shows a band structure subsequent to an interaction between oxidized MXene and CO₂ molecules for the chemimemristor device of Embodiment 2.

Referring to FIG. 9, the band structure subsequent to an interaction between CO₂ molecule and the structure of Ti₃C₂(OH)₂ is shown. Prior to the adsorption of CO₂ (red), Ti₃C₂(OH)₂ has metallic properties to exhibit a continuous band structure near the Fermi level (E_{f}), but subsequent to the adsorption of CO₂ (blue), electron states are rearranged to exhibit a pronounced change around the Fermi level. This shows that an interaction between CO₂ and Ti₃C₂(OH)₂ changes the electronic structure to affect the electrical properties of the device.

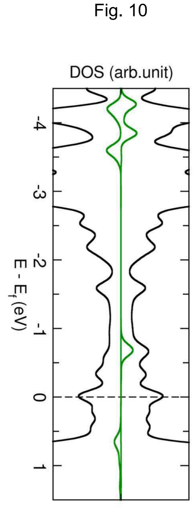

FIG. 10 shows results of projected density of states (DOS) for the chemimemristor device of Embodiment 2 before and after the adsorption of oxidized MXene and CO₂ molecules.

Referring to FIG. 10, in pure Ti₃C₂(OH)₂ (black), electron states exist near the Fermi level, but after CO₂ is adsorbed (green), it can be seen that new electron states are formed to change the charge density. Such a change in density of states indicates that the electrical properties of Ti₃C₂(OH)₂ are controlled by an interaction with CO₂.

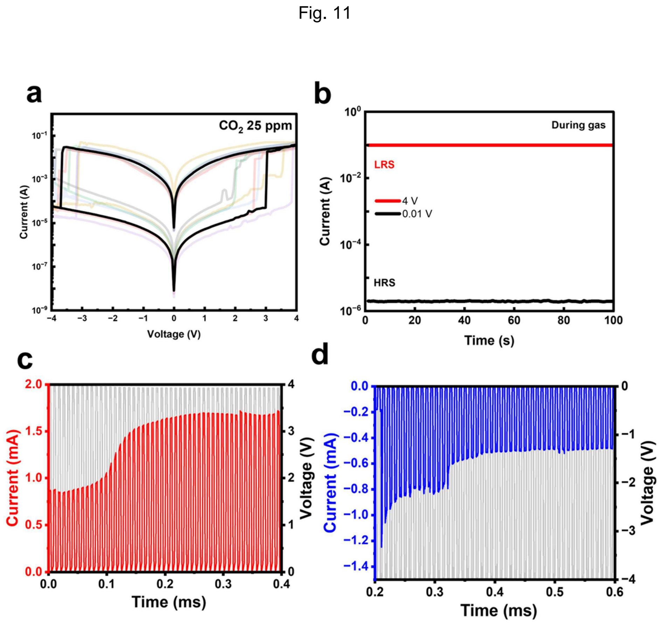

FIG. 11 shows results of (a) I-V curve, (b) data retention time, (c) short-term potentiation (STP), and (d) short-term depression (STD) for the chemimemristor device of Embodiment 2.

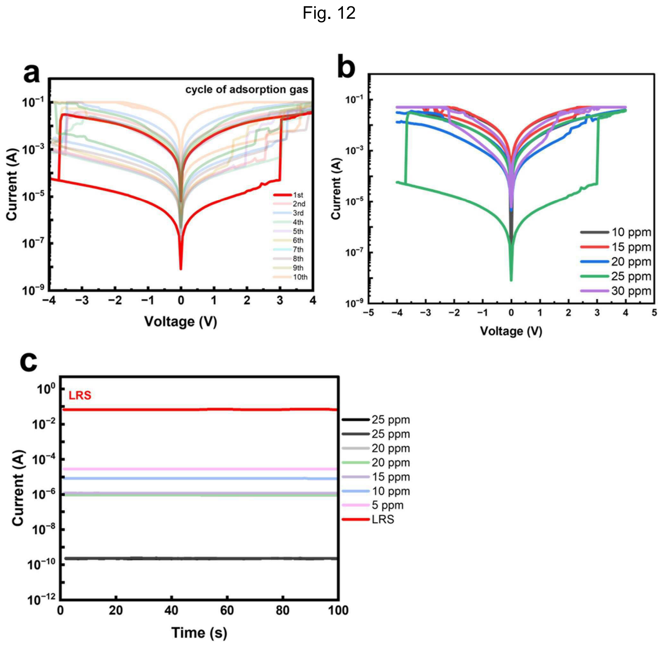

FIG. 12 shows results of (a) I-V characteristics according to CO₂ gas adsorption cycle, (b) I-V characteristics according to a concentration of CO₂, and (c) data retention characteristics (retention time) according to a concentration of CO₂ for the chemimemristor device of Embodiment 2.

Referring to FIGS. 11 and 12, it can be seen that the oxidized MXene device increases, when exposed to 25 ppm CO₂ gas, the conductance of the device to perform a gas sensing function ((a) of FIG. 11), exhibits data retention characteristics (retention time) in which the device maintains a high resistance state (HRS) and a low resistance state (LRS) even during the exposure to CO₂ gas ((b) of FIG. 11), and exhibits, when exposed to CO₂ gas, short-term potentiation (STP) and short-term depression (STD) characteristics, respectively, to implement neuromorphic behaviors that mimic synaptic plasticity ((c) and (d) of FIG. 11). In addition, I-V characteristics as CO₂ gas concentration and adsorption cycles proceed multiple times show that the device operates stably even with repeated gas adsorption and desorption ((a) of FIG. 12), and by checking I-V characteristics at various concentrations of CO₂ (10, 15, 20, 25 and 30 ppm), it can be seen that the conductance improves step-by-step as the concentration of CO₂ increases ((b) of FIG. 12). These results indicate that a single device can exhibit multiple levels of conductance, which means that multi-level information processing characteristics can be implemented. In addition, it can be seen that the higher the CO₂ gas concentration, the longer the conductance retention time ((c) of FIG. 12). Through this, it can be seen that the oxidized MXene-based chemimemristor device implements conductance changes and synaptic plasticity depending on the CO₂ gas concentration and exposure state, and has excellent repetitive gas detection and data retention characteristics.

As described above, the embodiments of the present disclosure have been described with reference to the accompanying drawings, but it will be apparent to those skilled in the art to which the disclosure pertains that the disclosure can be embodied in other specific forms without departing from the concept and essential characteristics thereof. Therefore, it should be understood that the foregoing embodiments are merely illustrative but not restrictive in all aspects.

## Claims

1. A method for manufacturing oxidized MXene, the method comprising:
etching a Mₙ₊₁AXₙ phase compound to manufacture Mₙ₊₁XₙF phase multilayer MXene (S01); and
immersing the MXene in an aqueous hydrogen peroxide solution to form OH functional groups on a surface of the MXene through an oxidation reaction and then drying the same to manufacture oxidized MXene (Mₙ₊₁Xₙ(OH)₂) (S02),
wherein M is Ti, Zr, Hf, V, Nb, Ta, Cr, Mo, W or two or more transition metals thereof, A is Al, Ga, In, Tl, Si, Ge, Sn, Pb, P, As, Sb or a combination thereof, X is C, N or a combination thereof, and n is 1, 2 or 3.

2. The method of claim 1, wherein in the step S02, through the oxidation reaction, an active reaction site capable of adsorbing CO₂ molecules is formed.

3. The method of claim 1, wherein in the step S02, forming, by the oxidation reaction, transition metal oxide particles represented by the chemical formula MO₂ on part of a surface of the oxidized MXene is included.

4. The method of claim 1, wherein a concentration of the aqueous hydrogen peroxide solution is 0.01 wt% to 0.5 wt%.

5. A chemimemristor device comprising:
a gas sensing layer comprising oxidized MXene manufactured according to any one of claims 1 to 4, and having an active reaction site capable of adsorbing CO₂ molecules; and
at least one conductive electrode in contact with a portion of the gas sensing layer.

6. The chemimemristor device of claim 5, wherein the active reaction site comprises carbon (C), titanium (Ti) and hydroxyl group (OH) sites of the oxidized MXene, and
wherein an order of high adsorption energy with CO₂ molecules at the active reaction site is carbon (C), titanium (Ti), and hydroxy group (OH) sites.

7. The chemimemristor device of claim 5, wherein an electrical conductance of the device reversibly changes according to a concentration of CO₂ gas.

8. The chemimemristor device of claim 5, wherein the device forms a multi-level conducting state according to the concentration of CO₂ gas.

9. The chemimemristor device of claim 5, wherein the device has an expanded memory window and an increased ON/OFF ratio when exposed to CO₂ gas.

10. The chemimemristor device of claim 5, wherein the device operates as a neuromorphic system by mimicking short-term potentiation (STP) and short-term depression (STD) characteristics during CO₂ gas adsorption.
